# EUROPÄISCHE PATENTSCHRIFT

(11) **EP 1 247 798 B1**
(45) Veröffentlichungstag und Bekanntmachung des Hinweises auf die Patenterteilung: **02.06.2004**
(21) Anmeldenummer: 02006614.8
(22) Anmeldetag: 25.03.2002
(51) Int. Cl.: C07C 201/08

(54) **Kontinuierliches isothermes Verfahren zur Herstellung von Mononitrotoluolen in Gegenwart vor Phosphorsäure**
Continuous isothermal process for the preparation of mononitrotoluene in the presence of phosphoric acid
Procédé en continu isotherme pour la préparation de mononitrotoluène en présence d'acide phosphorique

(30) Priorität: 06.04.2001 DE 10117207
(43) Veröffentlichungstag der Anmeldung: 09.10.2002
(73) Patentinhaber: Bayer Chemicals AG, 51368 Leverkusen (DE)
(72) Erfinder: Gotta, Matthias, Dr., 51061 Köln (DE); Demuth, Ralf, Dr., 40724 Hilden (DE); Zirngiebl, Eberhard, Dr., 51061 Köln (DE); Weber, Hans-Martin, Dr., 51373 Leverkusen (DE); Ronge, Georg, Dr., 40549 Düsseldorf (DE)

(56) Entgegenhaltungen:
- EP-A- 0 156 199
- EP-A- 1 205 468
- DE-A- 1 643 600

## Beschreibung

Die vorliegende Erfindung betrifft ein kontinuierliches isothermes Verfahren zur Herstellung von Mononitrotoluolen unter Verwendung von Mischungen aus Schwefelsäure und Phosphorsäure. Die dabei anfallende Abfallsäure wird einer verfahrensintegrierten Aufkonzentrierung unterworfen und anschließend in das Verfahren rückgeführt.

Mononitrotoluole sind wichtige Zwischenprodukte für die Herstellung von optischen Aufhellern, Pflanzenschutzmitteln und Pharmaprodukten. Sie können großtechnisch beispielsweise durch isotherme Nitrierung von Toluol hergestellt werden. Hierbei wird Toluol mit einer Mischung aus Schwefelsäure und Salpetersäure (Mischsäure, Nitriersäure) umgesetzt (Kirk-Othmer, Encyclopedia of Chemical Technology Vol. 17, 4^{th} Edition 1996, "Nitration" und "Nitrobenzenes and Nitrotoluene").

Bei Nitrierungen in reiner Schwefelsäure wird üblicherweise ein Verhältnis o-Nitrotoluol zu para-Nitrotoluol von ca. 1.65 erhalten (Albright&Hanson, Industrial and Laboratory Nitrations, 1976, S. 300 - 312). Der Ersatz der Schwefelsäure durch reine Phosphorsäure führt zu einer Verschiebung dieses Verhältnisses hin zu einem größeren Anteil des para-Isomers (Olah et al., Methods and Mechanisms, 1989, S. 15-18). Auf Grund der geringeren Säurestärke der Phosphorsäure gegenüber der Schwefelsäure kommt es jedoch bei deren Einsatz üblicherweise zu einer starken Verlangsamung der Reaktion. Zur Erreichung vergleichbarer Reaktionsgeschwindigkeiten wird daher der Einsatz hochkonzentrierter Polyphosphorsäuren empfohlen, herstellbar aus der Zumischung von P₂O₅ zu ortho-Phosphorsäure.

DE 164 36 00 A bewertet diese Vorgehensweise, indem auf die mit dem Einsatz von Polyphosphorsäuren verbundenen Probleme hinsichtlich deren Korrosivität gegenüber technisch gebräuchlichen Werkstoffen verwiesen wird.

In DE 164 36 00 A werden daraufhin Verfahrensparameter für die Nitrierung von Toluol in Phosphorsäure offenbart, wobei der Einsatz einer 100,8 %igen Phosphorsäure zu einem o/p-Nitrotoluol-Verhältnis von 0,90 führt. Die in den Beispielen verwendeten hohen Überschüsse an Mischsäure und die gewährten langen Reaktionszeiten zeigen jedoch dem Fachmann eine niedrige Raum-Zeit-Ausbeute an, die eine wirtschaftliche Nutzung des vorgestellten Verfahrens zweifelhaft erscheinen lassen.

Typischerweise wird in DE 164 36 00 A am Reaktorausgang eine ca. 97 %ige Phosphorsäure erhalten. Die zur Rückführung dieser Abfallsäure nötige Aufkonzentrierung erfordert eine Verdampfung des Reaktionswassers bei Temperaturen von ca. 180°C bei einem Druck von ca. 20 mbar, wie sich dem Siedediagramm des Systems H₂O-H₃PO₄ entnehmen lässt. Diese Parameter lassen sich nur mittels eines erheblichen Aufwandes sowohl hinsichtlich der verwendeten Werkstoffe als auch hinsichtlich der apparativen Anforderungen verwirklichen und stehen somit einer wirtschaftlich vorteilhaften Anwendung von reiner Phosphorsäure als Mischsäurekomponente entgegen. So lassen beispielsweise die Isokorrosionskurven von Email bei diesen Verfahrensbedingungen keine Beständigkeit gegenüber einer Phosphorsäure dieser Konzentration erwarten.

In DE 164 36 00 A wird zudem eine Verfahrensaltemative entwickelt, in der Schwefelsäure in einer Menge von bis zu 80 % der eingesetzten Phosphorsäure der Mischsäure zugesetzt wird. Dieser Zusatz von Schwefelsäure führt gegenüber der Verfahrensweise in reiner Phosphorsäure nicht zu einer signifikanten Verschiebung des Isomerenverhältnisses hin zum ortho-Isomeren. Er gibt dennoch dem Fachmann keine Hinweise zur Lösung der bei Einsatz von reiner Phosphorsäure auftretenden Probleme zur Hand, da die drastischen Verfahrensbedingungen zur Abfallsäureaufkonzentrierung, die gegenüber den gebräuchlichen Werkstoffen erhöhte Korrosivität der Säure und die niedrige Raum-Zeit-Ausbeute weiterhin einer technischen Realisierung eines wirtschaftlich vorteilhaft zu nutzenden Verfahrens entgegenstehen.

Technische Verfahren zur Mononitrierung von Toluol unter Einsatz von Mischsäure sind mit dem Anfall von Abfallsäuren verbunden, die mit erheblichen Anteilen an organischen Verbindungen wie beispielsweise Dinitrotoluolen oder nitrierten Kresolen belastet sind und verfahrens- und kostenintensiv aufgearbeitet werden müssen.

So wurden beispielsweise im Falle der Nitrierung unter Zusatz reiner Schwefelsäure Verfahren entwickelt, die eine Schwefelsäureaufkonzentrierung beinhalten, wobei die aufkonzentrierte Schwefelsäure von Wasser und organischen Verbindungen befreit, anschließend in einem Kreislaufprozess wieder in die Nitrierreaktion zurückgeführt, und somit der Anfall von Abfallsäure vermieden wird.

In DE 195 39 205 A werden Verfahrensparameter für die Mononitrierung von Aromaten offenbart, wobei die Mischsäuren so an die Eigenschaften des zu nitrierenden Aromaten angepasst werden, dass eine ca. 70 %ige Abfallschwefelsäure entsteht. Weiterhin ist der Einsatz von teilkonzentrierten Abfallsäuren mit einer Schwefelsäurekonzentration zwischen 85 % und 92 % beschrieben.

In US 4,772,757 ist ein Verfahren zur Herstellung von Nitrobenzol beschrieben, wobei die anfallende Abfallsäure auf 75 bis 92 % aufkonzentriert und wieder in das Nitrierverfahren zurückgeführt wird. Da Toluol aufgrund der Methylgruppe im Vergleich zu Benzol deutlich oxidationsempfindlicher ist und bei der Nitrierung zur Bildung von Nebenprodukten neigt, ist bei der Übertragung von Reaktionsbedingungen zur Nitrierung von Benzol auf die Nitrierung von Toluol mit einer Erhöhung der Menge an unerwünschten Nebenprodukten zu rechnen.

Auf Grund der Löslichkeit von organischen Verbindungen in anorganischen Säuren reichem sich in aufkonzentrierten Abfallsäuren, die in die Nitrierreaktion zurückgeführt werden, organische Nebenprodukte wie Oxalsäure oder Benzoesäure an. Weiterhin kann es zu einer Anreicherung an Nitrosylschwefelsäure kommen. Die Zersetzung dieser Nebenprodukte und die dabei freiwerdende Zersetzungswärme kann zur unerwünschten Zersetzung des Reaktionsprodukts Nitrotoluol führen.

Darüber hinaus ist eine Beeinträchtigung der Raum-Zeit-Ausbeute gegeben, da die organischen Nebenprodukte mit der eingesetzten Salpetersäure in oxidativen Abbaureaktionen reagieren können und somit ein Teil der Salpetersäure für die eigentliche Nitrierung nicht mehr zur Verfügung steht.

Es bestand daher Bedarf an einem kontinuierlichen isothermen Verfahren zur Herstellung von Mononitrotoluol mit einer erhöhten Ausbeute an para-Nitrotoluol durch Einsatz von Mischungen aus Schwefel- und Phosphorsäure, welches eine kostengünstige Aufkonzentrierung der Abfallsäure mit anschließender Rückführung in die Nitrierreaktion im Sinne eines Kreislaufprozesses erlaubt, ohne dass sich in der aufkonzentrierten Säure organische Nebenprodukte anreichern. Zusätzlich ist der Einsatz einer verdünnten Salpetersäure von 60-70 wt% erstrebenswert, da dieser zu einer deutlichen Kostenreduktion im Vergleich zu dem Einsatz von hochkonzentrierter Salpetersäure führt.

Es wurde ein kontinuierliches Verfahren zur Herstellung von Mononitrotoluolen durch Umsetzung von Toluol mit Salpetersäure, Schwefelsäure und Phosphorsäure unter isothermen Reaktionsbedingungen gefunden, das dadurch gekennzeichnet ist, dass Mischungen aus Schwefelsäure und Phosphorsäure als Mischsäurekomponente eingesetzt werden, die sich aus 45 bis 80 % Schwefelsäure, 9 bis 45 % Phosphorsäure und 5 bis 15 % Wasser zusammensetzen.

Eine besonders bevorzugte Ausführungsform des erfindungsgemäßen Verfahrens ist dadurch gekennzeichnet, dass Mischungen aus Schwefelsäure und Phosphorsäure als Mischsäurekomponente eingesetzt werden, die sich aus 64 bis 78 % Schwefelsäure, 10 bis 27 % Phosphorsäure und 8 bis 13 % Wasser zusammensetzen.

Eine besondere Ausführungsform des erfindungsgemäßen Verfahrens ist dadurch gekennzeichnet, dass in einem nachfolgenden Schritt die Mischsäurekomponente und 60 bis 70 %ige Salpetersäure und Toluol in den Reaktor eingespeist werden. Das erfindungsgemäße Verfahren ist dadurch gekennzeichnet, dass Mischsäurekomponente und 60-70 %ige Salpetersäure in einem Gewichtsverhältnis von 2:1 bis 4:1 bezogen auf Salpetersäure und Mischsäurekomponente und Toluol in einem Gewichtsverhältnis von 2:1 bis 4:1 bezogen auf Toluol in den Reaktor eingespeist werden.

Eine besonders bevorzugte Ausführungsform des erfindungsgemäßen Verfahrens ist dadurch gekennzeichnet, dass in einem nachfolgenden Schritt die Mischsäurekomponente und 60 bis 70 %ige Salpetersäure und Toluol in den Reaktor eingespeist werden. Das erfindungsgemäße Verfahren ist dadurch gekennzeichnet, dass Mischsäurekomponente und 60-70 %ige Salpetersäure in einem Gewichtsverhältnis von 2,4:1 bis 3,3:1 bezogen auf Salpetersäure und Mischsäurekomponente und Toluol in einem Gewichtsverhältnis von 2,4:1 bis 3,2:1 bezogen auf Toluol in den Reaktor eingespeist werden.

Eine besondere Ausführungsform des erfindungsgemäßen Verfahrens ist dadurch gekennzeichnet, dass in einem nachfolgenden Schritt am Reaktorausgang eine Trennung des Rohnitrotoluols von der Abfallsäure erfolgt.

Eine besondere Ausführungsform des erfindungsgemäßen Verfahrens ist dadurch gekennzeichnet, dass sich die Abfallsäure am Reaktorausgang aus 42 bis 70 % Schwefelsäure, 6 bis 37 % Phosphorsäure und 15 bis 28 % Wasser zusammensetzt. Die Abfallsäure ist nahezu frei von Salpetersäure und kann zudem organische Verbindungen wie beispielsweise Dinitrotoluole oder nitrierte Kresole und gegebenenfalls salpetrige Säure enthalten.

Eine besonders bevorzugte Ausführungsform des erfindungsgemäßen Verfahrens ist dadurch gekennzeichnet, dass sich die Abfallsäure am Reaktorausgang aus 54 bis 67 % Schwefelsäure, 7 bis 22 % Phosphorsäure und 18 bis 27 % Wasser zusammensetzt.

Eine besondere Ausführungsform des erfindungsgemäßen Verfahrens ist dadurch gekennzeichnet, dass in einem nachfolgenden Schritt die Abfallsäure einer einstufigen Aufkonzentrierung auf eine Zusammensetzung von 45 bis 80 % Schwefelsäure, 9 bis 45 % Phosphorsäure und 5 bis 15 % Wasser unterzogen wird.

Eine besonders bevorzugte Ausführungsform des erfindungsgemäßen Verfahrens ist dadurch gekennzeichnet, dass die Abfallsäure einer einstufigen Aufkonzentrierung auf eine Zusammensetzung von 64 bis 78 % Schwefelsäure, 10 bis 27 % Phosphorsäure und 8 bis 13 % Wasser unterzogen wird.

Eine besondere Ausführungsform des erfindungsgemäßen Verfahrens ist dadurch gekennzeichnet, dass in einem nachfolgenden Schritt die aufkonzentrierte Abfallsäure in einer Kreisführung wieder in die Nitrierreaktion zurückgeführt wird.

Eine besondere Ausführungsform des erfindungsgemäßen Verfahrens ist dadurch gekennzeichnet, dass
a) Mischungen aus Schwefelsäure und Phosphorsäure als Mischsäurekomponente eingesetzt werden, die sich aus 45 bis 80 % Schwefelsäure, 9 bis 45 % Phosphorsäure und 5 bis 15 % Wasser zusammensetzen,
b) die Mischsäurekomponente, 60 bis 70 %ige Salpetersäure und Toluol in einen Reaktor eingespeist werden, wobei Mischsäurekomponente und Salpetersäure im Gewichtsverhältnis von 2:1 bis 4:1 bezogen auf Salpetersäure und Mischsäurekomponente und Toluol in einem Gewichtsverhältnis von 2:1 bis 4:1 bezogen auf Toluol stehen,
c) am Reaktorausgang eine Trennung des Rohnitrotoluols von der Abfallsäure erfolgt,
d) die Abfallsäure, die sich aus 42 bis 70 % Schwefelsäure, 6 bis 37 % Phosphorsäure und 15 bis 28 % Wasser zusammensetzt, in einer einstufigen Aufkonzentrierung auf eine Zusammensetzung von 45 bis 80 % Schwefelsäure, 9 bis 45 % Phosphorsäure und 5 bis 15 % Wasser aufkonzentriert wird und
e) die aufkonzentrierte Abfallsäure in einer Kreisführung wieder in die Nitrierreaktion zurückgeführt wird.

Eine besonders bevorzugte Ausführungsform des erfindungsgemäßen Verfahrens ist dadurch gekennzeichnet, dass
a) Mischungen aus Schwefelsäure und Phosphorsäure als Mischsäurekomponente eingesetzt werden, die sich aus 64 bis 78 % Schwefelsäure, 10 bis 27 % Phosphorsäure und 8 bis 13 % Wasser zusammensetzen,
b) die Mischsäurekomponente, 60 bis 70 %ige Salpetersäure und Toluol in einen Reaktor eingespeist werden, wobei Mischsäurekomponente und Salpetersäure im Gewichtsverhältnis von 2,4:1 bis 3,3:1 bezogen auf Salpetersäure und Mischsäurekomponente und Toluol in einem Gewichtsverhältnis von 2,4:1 bis 3,2: bezogen auf Toluol stehen,
c) am Reaktorausgang eine Trennung des Rohnitrotoluols von der Abfallsäure erfolgt,
d) die Abfallsäure, die sich aus 54 bis 67 % Schwefelsäure, 7 bis 22 % Phosphorsäure und 18 bis 27 % Wasser zusammensetzt, in einer einstufigen Aufkonzentrierung auf eine Zusammensetzung von 64 bis 78 % Schwefelsäure, 10 bis 27 % Phosphorsäure und 8 bis 13 % Wasser aufkonzentriert wird und
e) die aufkonzentrierte Abfallsäure in einer Kreisführung wieder in die Nitrierreaktion zurückgeführt wird.

Das erfindungsgemäße Verfahren führt gegenüber der in der Technik gebräuchlichen Nitrierung in reiner Schwefelsäure überraschenderweise zu einem erhöhten Anteil an para-Nitrotoluol bei einem bereits geringen Anteil an Phosphorsäure als Mischsäurekomponente. Para-Nitrotoluol hat besonders Interesse als Zwischenprodukt für die Herstellung von optischen Aufhellern, von Wirkstoffen für Pharma und Agro und von Farb- und Riechstoffen.

Im erfindungsgemäßen Verfahren wird in vorteilhafter Weise eine verdünnte Salpetersäure eingesetzt, wodurch das Verfahren besonders kostengünstig betrieben werden kann. Trotz verhältnismäßig hoher Wassermengen, die durch den Einsatz von verdünnter Salpetersäure bedingt sind, läuft die Reaktion mit hohen Reaktionsgeschwindigkeiten ab. Die Reaktionsgeschwindigkeit hängt im erfindungsgemäßen Verfahren im Wesentlichen von der Konzentration der eingesetzten Schwefelsäure ab. Die eingesetzte Phosphorsäuremenge dient somit in erster Linie zur Steuerung des Isomerenverhältnisses.

Im erfindungsgemäßen Verfahren werden vorzugsweise 0,98 bis 1,1 Äquivalente Toluol bezogen auf ein Äquivalent Salpetersäure eingesetzt, besonders bevorzugt werden 1,01 bis 1,05 Äquivalente Toluol bezogen auf ein Äquivalent Salpetersäure eingesetzt. Auch bei Toluolüberschuss bezogen auf Salpetersäure tritt im erfindungsgemäßen Verfahren überraschenderweise keine Zunahme an Verunreinigungen auf.

Die im erfindungsgemäßen Verfahren eingesetzten Edukte Salpetersäure, Schwefelsäure, Phosphorsäure und Toluol werden vorzugsweise mit den in der Technik bekannten Mischorganen intensiv gemischt. Als Mischorgane können beispielsweise statische Mischer, Pumpen, Düsen, Rührer oder Kombinationen der genannten Mischorgane eingesetzt werden.

Das erfindungsgemäße Verfahren wird kontinuierlich unter isothermen Bedingungen in einem Reaktor durchgeführt. Als Reaktoren werden vorzugsweise kommerziell erhältliche Reaktoren wie beispielsweise Rohrreaktoren, Schlaufenreaktoren, Rührkessel oder auch Kombinationen von Schlaufenreaktoren und Rührkesseln eingesetzt. In einer weiteren bevorzugten Ausführungsform wird das erfindungsgemäße Verfahren in mehrstufigen Reaktorkaskaden durchgeführt.

Das erfindungsgemäße Verfahren wird unter isothermen Bedingungen durchgeführt, wobei die Reaktionstemperatur vorzugsweise im Bereich von 20 bis 80°C, besonders bevorzugt im Bereich von 30 bis 70°C und ganz besonders bevorzugt im Bereich von 40 bis 65°C liegt.

Die Trennung des Rohnitrotoluols von der Abfallsäure wird vorzugsweise mit Hilfe eines statischen Scheiders oder eines Separators durchgeführt. Hierbei finden dem Fachmann bekannte Methoden der statischen oder dynamischen Phasentrennung Anwendung, wie z.B. Separatoren oder Scheideflaschen mit und ohne Einbauten.

Durch die im erfindungsgemäßen Verfahren durchgeführte Aufkonzentrierung der Abfallsäure wird diese weitgehend von Wasser und organischen Verbindungen befreit, wobei die organischen Verbindungen entweder aus der Abfallsäure entfernt werden oder so zerstört werden, dass leichtflüchtige Verbindungen wie beispielsweise CO₂ entstehen, die aus der Abfallsäure ausgetragen werden.

Die einstufige Aufkonzentrierung wird vorzugsweise in einem Verdampfer durchgeführt. Um die erfindungsgemäße Zusammensetzung der Schwefelsäure-Phosphorsäure-Mischung zu erhalten, wird der Verdampfer vorzugsweise bei einem Druck von 30 bis 300 mbar, besonders bevorzugt von 60 bis 200 mbar und ganz besonders bevorzugt von 80 bis 150 mbar betrieben. Die Temperatur der Abfallsäure im Austritt des Verdampfers beträgt dabei vorzugsweise 100 bis 200°C, besonders bevorzugt 150 bis 190°C und ganz besonders bevorzugt 155 bis 185°C. Die Temperatur der ablaufenden aufkonzentrierten Abfallsäure wird vorzugsweise dazu genutzt, um in einem Gegenstromwärmetauscher die in den Verdampfer strömende Abfallsäure aufzuheizen. Hierbei wird die in den Verdampfer strömende Abfallsäure durch die Gegenstromführung vorzugsweise so weit aufgeheizt, dass diese bei Verdampferdruck überhitzt ist und somit ein Teil des Wassers und geringe Teile der Säure ohne zusätzliche Wärmezufuhr abdampfen (Flash-Verdampfung).

Für die einstufige Aufkonzentrierung im erfindungsgemäßen Verfahren wird vorzugsweise ein auch kommerziell erhältlicher, einstufig destillierender und in der Länge kaskadierter Verdampfer mit Tantalrohrbündel verwendet, bei dem vom Eintritt kommend mit jeder Kaskade die Säurekonzentration erhöht wird, so dass in den ersten Kaskaden eine relativ niederkonzentrierte Säure vorliegt. Vorteil der niedrigen Konzentration in den ersten Kaskaden ist einerseits, dass der Siedepunkt noch niedrig ist und somit eine hohe treibende Temperaturdifferenz für den Wärmeübergang vorliegt (kleinerer Verdampfer) und andererseits, dass bei niedrigen Säurekonzentrationen eventuell in der Abfallsäure vorliegende Nitrosylschwefelsäure aus der Reaktion gut entfernbar ist. Somit wird durch die Verwendung eines einstufigen kaskadierten Verdampfers im erfindungsgemäßen Verfahren das Ausblasen der Nitrosylschwefelsäure mit Schwefeldioxid und somit ein zusätzlicher Verfahrensschritt vermieden.

Vorzugsweise wird ein Abtriebteil eingesetzt, um eine besonders gute Reduzierung des Gehalts an organischen Verbindungen und/oder des Gehalts an Nitrosylschwefelsäure zu erreichen. Als Abtriebsteil wird ein mit Destillationseinbauten versehener Destillationskolonnenabschnitt bezeichnet, auf den von oben die bei Verdampferdruck flüssige oder etwas überhitzte Abfallsäure geleitet wird und der im Gegenstrom von unten mit dem aus dem Verdampfer aufsteigenden Dampf betrieben wird. Als Destillationseinbauten kommen im Abtriebsteil die dem Fachmann bekannten Kolonneneinbauten wie beispielsweise Böden, Packungen und Füllkörper zur Anwendung. In einer bevorzugten Ausführungsform werden druckverlustarme Destillationseinbauten wie geordnete Packungen oder Füllkörper eingesetzt. Die Verweilzeit im Abtriebsteil bei gleichzeitig geringer Säurekonzentration führt zusammen mit dem durch die Destillationseinbauten intensivierten Stoffaustausch in vorteilhafter Weise zu einer schnellen Zersetzung und Abtrennung von organischen und anorganischen Verbindungen.

Das im erfindungsgemäßen Verfahren erhaltene Rohnitrotoluol enthält in der Regel weniger als 0,5 % dinitrierte Verbindungen und weniger als 0,8 % Dinitrokresole.

Im erfindungsgemäßen Verfahren kann ein Teilstrom entnommen werden, um gegebenenfalls das Aufkonzentrieren von Nebenprodukten im Säurekreislauf zu vermeiden.

Im erfindungsgemäßen Verfahren besteht die Möglichkeit, auch Toluol mit einem geringen Gehalt an Dinitrotoluolen und nitrierten Kresolen als Ausgangsmaterial zu verwenden.

Im erfindungsgemäßen Verfahren besteht die Möglichkeit, zur Verhinderung des Angriffs der sich aus Schwefelsäure-Phosphorsäure zusammensetzenden Mischsäurekomponente auf die verwendeten Werkstoffe diese mit bis zu 2000 ppm Wasserglas zu dotieren.

### Beispiele

### Beispiel 1

In einer Miniplant-Anlage wurden einer Rührkesselkaskade pro Stunde 1,03 kg einer Säure der Zusammensetzung 75 % H₂SO₄, 13 % H₃PO₄ und 12 % H₂O, 0,33 kg 68 %ige Salpetersäure und 0,33 kg Toluol zugeführt. Die Temperatur in den Rührkesseln betrug ca. 45°C. Nach Beendigung der Reaktion wurde das Rohnitrotoluol von der Abfallsäure mittels eines statischen Scheiders abgetrennt. Die Abfallsäure wurde über einen Vorwärmer einem Verdampfer zugeführt und bei 100 mbar und 170°C auf 75 % H₂SO₄, 13 % H₃PO₄ und 12 % H₂O aufkonzentriert, wobei organische Verbindungen abdestilliert oder zerstört wurden. Die aufkonzentrierte Abfallsäure wird der Nitrierreaktion erneut zugeführt.

Das erhaltene Rohnitrotoluol wies folgende Zusammensetzung auf:
2,23 % Toluol, 55,88 % ortho-Nitrotoluol, 4,39 % meta-Nitrotoluol, 37,16 % para-Nitrotoluol, 0,31 % Dinitrotoluol und 0,26 % Dinitrokresol.
Ortho-Nitrotoluol/para-Nitrotoluol = 1,50.

### Beispiel 2

In einer Miniplant-Anlage wurden einer Rührkesselkaskade pro Stunde 1,03 kg einer Säure der Zusammensetzung 54 % H₂SO₄, 38 % H₃PO₄ und 8 % H₂O, 0,33 kg 68 %ige Salpetersäure und 0,33 kg Toluol zugeführt. Die Temperatur in den Rührkesseln betrug ca. 45°C. Nach Beendigung der Reaktion wurde das Rohnitrotoluol von der Abfallsäure mittels eines statischen Scheiders abgetrennt. Die Abfallsäure wurde über einen Vorwärmer einem Verdampfer zugeführt und bei 100 mbar und 170°C auf 54 % H₂SO₄, 38 % H₃PO₄ und 8 % H₂O aufkonzentriert, wobei organische Verbindungen abdestilliert oder zerstört wurden. Die aufkonzentrierte Abfallsäure wird der Nitrierreaktion erneut zugeführt.

Das erhaltene Rohnitrotoluol wies folgende Zusammensetzung auf:
2,44 % Toluol, 54,99 % ortho-Nitrotoluol, 4,42 % meta-Nitrotoluol, 37,67 % para-Nitrotoluol, 0,19 % Dinitrotoluol und 0,29 % Dinitrokresol.
Ortho-Nitrotoluol/para-Nitrotoluol = 1,46.

### Beispiel 3

In einer Miniplant-Anlage wurden einer Rührkesselkaskade pro Stunde 1,03 kg einer Säure der Zusammensetzung 67 % H₂SO₄, 22 % H₃PO₄ und 11 % H₂O, 0,33 kg 68 %ige Salpetersäure und 0,33 kg Toluol zugeführt. Die Temperatur in den Rührkesseln betrug ca. 45°C. Nach Beendigung der Reaktion wurde das Rohnitrotoluol von der Abfallsäure mittels eines statischen Scheiders abgetrennt. Die Abfallsäure wurde über einen Vorwärmer einem Verdampfer zugeführt und bei 100 mbar und 168°C auf 67 % H₂SO₄, 22 % H₃PO₄ und 11 % H₂O aufkonzentriert, wobei organische Verbindungen abdestilliert oder zerstört wurden. Die aufkonzentrierte Abfallsäure wird der Nitrierreaktion erneut zugeführt.

Das erhaltene Rohnitrotoluol wies folgende Zusammensetzung auf:
2,25 % Toluol, 55,66 % ortho-Nitrotoluol, 4,36 % meta-Nitrotoluol, 37,24 % para-Nitrotoluol, 0,23 % Dinitrotoluol und 0,26 % Dinitrokresol.
Ortho-Nitrotoluol/para-Nitrotoluol = 1,49.

### Vergleichsbeispiel 1

In einer Miniplant-Anlage wurden einer Rührkesselkaskade pro Stunde 0,80 kg 87,7 %ige Schwefelsäure, 0,31 kg 67 %ige Salpetersäure und 0,32 kg Toluol zugeführt. Die Temperatur in den Rührkesseln betrug ca. 40°C. Nach Beendigung der Reaktion wurde das Rohnitrotoluol von der Abfallschwefelsäure mittels eines statischen Scheiders abgetrennt. Die Abfallschwefelsäure wurde über einen Vorwärmer einem Verdampfer zugeführt und bei 100 mbar und 168°C auf 87,7 % aufkonzentriert, wobei organische Verbindungen abdestilliert oder zerstört wurden. Die Abfallschwefelsäure kann der Nitrierreaktion erneut zugeführt werden.

Das erhaltene Rohnitrotoluol wies folgende Zusammensetzung auf:
3,27 % Toluol, 57,58 % ortho-Nitrotoluol, 4,13 % meta-Nitrotoluol, 34,68 % para-Nitrotoluol, 0,08 % Dinitrotoluol und 0,38 % Dinitrokresol.
Ortho-Nitrotoluol/para-Nitrotoluol = 1,66

### Vergleichsbeispiel 2

Einer Kaskade aus mehreren Schlaufenreaktoren wurden stündlich 3000 1 ca. 97 %iges Toluol, welches geringe Mengen nitrierter Toluole und Kresole enthielt, 3700 l 87 %ige Schwefelsäure und 1800 l 67 bis 68 %ige Salpetersäure zugeführt. Die Reaktoren wurden zwischen 43 und 47°C betrieben. Nach Beendigung der Reaktion wurde das Rohnitrotoluol von der Abfallschwefelsäure mittels eines Separators abgetrennt. Die Abfallschwefelsäure wurde bei ca. 170°C und 100 mbar wieder auf den Anfangswert von 87 % aufkonzentriert und in die Nitrierreaktion zurückgeführt.

Das erhaltene Rohnitrotoluol wies folgende Zusammensetzung auf:
4,13 % Toluol, 57,12 % ortho-Nitrotoluol, 4,18 % meta-Nitrotoluol, 34,17 % para-Nitrotoluol, 0,12 % Dinitrotoluol und 0,71 % Kresole.
Ortho-Nitrotoluol/para-Nitrotoluol = 1,67.

Im Vergleich zu den erfindungsgemäßen Beispielen wird bei vergleichbaren Werten für die allgemeinen Reaktionsbedingungen ein geringerer Anteil an para-Nitrotoluol bei sonst ähnlichem Nebenproduktspektrum gefunden.

## Patentansprüche

1. Kontinuierliches Verfahren zur Herstellung von Mononitrotoluolen durch Umsetzung von Toluol mit Salpetersäure, Schwefelsäure und Phosphorsäure unter isothermen Reaktionsbedingungen, **dadurch gekennzeichnet, dass** Mischungen aus Schwefelsäure und Phosphorsäure als Mischsäurekomponente eingesetzt werden, die sich aus 45 bis 80 % Schwefelsäure, 9 bis 45 % Phosphorsäure und 5 bis 15 % Wasser zusammensetzen.

2. Verfahren nach Anspruch 1, **dadurch gekennzeichnet, dass** die Mischsäurekomponente und 60 bis 70 %ige Salpetersäure und Toluol in den Reaktor eingespeist werden.

3. Verfahren nach den Ansprüchen 1 und 2, **dadurch gekennzeichnet, dass** am Reaktorausgang durch Flüssig-Flüssig-Phasentrennung eine Trennung des Rohnitrotoluols von der Abfallsäure erfolgt.

4. Verfahren nach den Ansprüchen 1 bis 3, **dadurch gekennzeichnet, dass** die Abfallsäure einer einstufigen Aufkonzentrierung auf eine Zusammensetzung von 45 bis 80 % Schwefelsäure, 9 bis 45 % Phosphorsäure und 5 bis 15 % Wasser unterzogen wird.

5. Verfahren nach den Ansprüchen 1 bis 4, **dadurch gekennzeichnet, dass** die aufkonzentrierte Abfallsäure in einer Kreisführung wieder in die Nitrierreaktion zurückgeführt wird.

6. Verfahren nach den Ansprüchen 1 bis 5, **dadurch gekennzeichnet, dass**
a) Mischungen aus Schwefelsäure und Phosphorsäure als Mischsäurekomponente eingesetzt werden, die sich aus 45 bis 80 % Schwefelsäure, 9 bis 45 % Phosphorsäure und 5 bis 15 % Wasser zusammensetzen,
b) die Mischsäurekomponente, 60 bis 70 %ige Salpetersäure und Toluol in einen Reaktor eingespeist werden,
c) am Reaktorausgang eine Trennung des Rohnitrotoluols von der Abfallsäure erfolgt,
d) die Abfallsäure in einer einstufigen Aufkonzentrierung auf eine Zusammensetzung von 45 bis 80 % Schwefelsäure, 9 bis 45 % Phosphorsäure und 5 bis 15 % Wasser aufkonzentriert wird und
e) die aufkonzentrierte Abfallsäure in einer Kreisführung wieder in die Nitrierreaktion zurückgeführt wird.

7. Verfahren nach den Ansprüchen 1 bis 6, **dadurch gekennzeichnet, dass** eine Mischsäurekomponente der Zusammensetzung 64 bis 78 % Schwefelsäure, 10 bis 27 % Phosphorsäure und 8 bis 13 % Wasser eingesetzt wird.

8. Verfahren nach den Ansprüchen 1 bis 7, **dadurch gekennzeichnet, dass** die Mischsäurekomponente und 60 bis 70 %ige Salpetersäure und Toluol in den Reaktor eingespeist werden.

9. Verfahren nach den Ansprüchen 1 bis 8, **dadurch gekennzeichnet, dass** eine 65 bis 68 %ige Salpetersäure eingesetzt wird.

10. Verfahren nach den Ansprüchen 1 bis 9, **dadurch gekennzeichnet, dass** 0,98 bis 1,1 Äquivalente Toluol, bezogen auf ein Äquivalent Salpetersäure eingesetzt werden.

11. Verfahren nach den Ansprüchen 1 bis 10, **dadurch gekennzeichnet, dass** 1,01 bis 1,05 Äquivalente Toluol, bezogen auf ein Äquivalent Salpetersäure eingesetzt werden.

12. Verfahren nach den Ansprüchen 1 bis 11, **dadurch gekennzeichnet, dass** die Zusammensetzung der Abfallsäure am Reaktorausgang 54 bis 67 % Schwefelsäure, 7 bis 22 % Phosphorsäure sowie 18 bis 27 % Wasser beträgt.

13. Verfahren nach den Ansprüchen 1 bis 12, **dadurch gekennzeichnet, dass** die Aufkonzentrierung in einem Verdampfer bei einem Druck von 30 bis 300 mbar und einer Temperatur von 100 bis 200°C durchgeführt wird.

14. Verfahren nach den Ansprüchen 1 bis 13, **dadurch gekennzeichnet, dass** die Aufkonzentrierung in einem kaskadierten Verdampfer durchgeführt wird.

15. Verfahren nach den Ansprüchen 1 bis 14, **dadurch gekennzeichnet, dass** der Verdampfer mit einem Abtriebteil betrieben wird.

16. Verfahren nach den Ansprüchen 1 bis 15, **dadurch gekennzeichnet, dass** die Reaktionstemperatur im Bereich von 20 bis 80°C liegt.

17. Verfahren nach den Ansprüchen 1 bis 16, **dadurch gekennzeichnet, dass** zur Verhinderung des Angriffs der Mischsäurekomponente auf verwendete Werkstoffe ein Zusatz von bis zu 2000 ppm Wasserglas erfolgen kann.

## Claims

1. Continuous process for preparing mononitrotoluenes by reacting toluene with nitric acid, sulphuric acid and phosphoric acid under isothermal reaction conditions, **characterized in that** mixtures of sulphuric acid and phosphoric acid are used as mixed acid component, which mixtures comprise 45 to 80% sulphuric acid, 9 to 45% phosphoric acid and 5 to 15% water.

2. Process according to Claim 1, **characterized in that** the mixed acid component and 60 to 70% strength nitric acid and toluene are fed into the reactor.

3. Process according to Claims 1 and 2, **characterized in that** the crude nitrotoluene is separated from the waste acid at the reactor outlet by liquid-liquid phase separation.

4. Process according to Claims 1 to 3, **characterized in that** the waste acid is subjected to a single-stage concentration to a composition of 45 to 80% sulphuric acid, 9 to 45% phosphoric acid and 5 to 15% water.

5. Process according to Claims 1 to 4, **characterized in that** the concentrated waste acid is recycled back to the nitration reaction in a circuit.

6. Process according to Claims 1 to 5, **characterized in that**
a) mixtures of sulphuric acid and phosphoric acid are used as mixed acid component, which mixtures comprise 45 to 80% sulphuric acid, 9 to 45% phosphoric acid and 5 to 15% water,
b) the mixed acid component, 60 to 70% strength nitric acid and toluene are fed into a reactor,
c) the crude nitrotoluene is separated from the waste acid at the reactor outlet,
d) the waste acid is concentrated in a single-stage concentration to a composition of 45 to 80% sulphuric acid, 9 to 45% phosphoric acid and 5 to 15% water and
e) the concentrated waste acid is recycled back to the nitration reaction in a circuit.

7. Process according to Claims 1 to 6, **characterized in that** a mixed acid component of composition 64 to 78% sulphuric acid, 10 to 27% phosphoric acid and 8 to 13% water is used.

8. Process according to Claims 1 to 7, **characterized in that** the mixed acid component and 60 to 70% strength nitric acid and toluene are fed into the reactor.

9. Process according to Claims 1 to 8, **characterized in that** a 65 to 68% strength nitric acid is used.

10. Process according to Claims 1 to 9, **characterized in that** 0.98 to 1.1 equivalents of toluene are used, based on one equivalent of nitric acid.

11. Process according to Claims 1 to 10, **characterized in that** 1.01 to 1.05 equivalents of toluene are used, based on one equivalent of nitric acid.

12. Process according to Claims 1 to 11, **characterized in that** the composition of the waste acid at the reactor outlet is 54 to 67% sulphuric acid, 7 to 22% phosphoric acid and 18 to 27% water.

13. Process according to Claims 1 to 12, **characterized in that** the concentration in an evaporator is performed at a pressure of 30 to 300 mbar and at a temperature of 100 to 200°C.

14. Process according to Claims 1 to 13, **characterized in that** the concentration is performed in a cascade-type evaporator.

15. Process according to Claims 1 to 14, **characterized in that** the evaporator is operated with a stripping section.

16. Process according to Claims 1 to 15, **characterized in that** the reaction temperature is in the range from 20 to 80°C.

17. Process according to Claims 1 to 16, **characterized in that**, to prevent the mixed acid component from attacking materials used, up to 2 000 ppm of water glass can be added.

## Revendications

1. Procédé continu de préparation de mononitrotoluènes par mise en réaction de toluène avec de l'acide nitrique, de l'acide sulfurique et de l'acide phosphorique dans des conditions de réaction isothermiques, **caractérisé en ce que** des mélanges d'acide sulfurique et d'acide phosphorique sont utilisés comme composant acide mixte et se composent de 45 à 80% d'acide sulfurique, 9 à 45% d'acide phosphorique et 5 à 15% d'eau.

2. Procédé selon la revendication 1, **caractérisé en ce que** le composant acide mixte, de l'acide nitrique à 60 à 70% et du toluène sont chargés dans le réacteur.

3. Procédé selon les revendications 1 et 2, **caractérisé en ce qu'**une séparation du nitrotoluène brut des déchets acides est effectuée par séparation des phases liquide ― liquide à la sortie du réacteur.

4. Procédé selon les revendications 1 à 3, **caractérisé en ce que** les déchets acides sont soumis à une concentration en une seule étape jusqu'à une composition de 45 à 80% d'acide sulfurique, 9 à 45% d'acide phosphorique et 5 à 15% d'eau.

5. Procédé selon les revendications 1 à 4, **caractérisé en ce que** les déchets acides concentrés sont recyclés dans la réaction de nitration.

6. Procédé selon les revendications 1 à 5, caractérisé en ce
a) qu'on utilise comme composant acide mixte des mélanges d'acide sulfurique et d'acide phosphorique qui se composent de 45 à 80% d'acide sulfurique, 9 à 45% d'acide phosphorique et 5 à 15% d'eau,
b) le composant acide mixte, de l'acide nitrique à 60 à 70% et du toluène sont chargés dans un réacteur,
c) une séparation du nitrotoluène brut et des déchets acides intervient à la sortie du réacteur,
d) les déchets acides sont concentrés dans une concentration en une étape jusqu'à une composition de 45 à 80% d'acide sulfurique, 9 à 45% d'acide phosphorique et 5 à 15% d'eau et
e) les déchets acides concentrés sont recyclés dans la réaction de nitration.

7. Procédé selon les revendications 1 à 6, **caractérisé en ce que** la composant acide mixte utilisé est composé de 64 à 78% d'acide sulfurique, 10 à 27% d'acide phosphorique et 8 à 13 % d'eau.

8. Procédé selon les revendications 1 à 7, **caractérisé en ce que** le composant acide mixte et de l'acide nitrique à 60 à 70 % et du toluène sont chargés dans le réacteur.

9. Procédé selon les revendications 1 à 8, **caractérisé en ce que** de l'acide nitrique à 65 à 68% est utilisé.

10. Procédé selon les revendications 1 à 9, **caractérisé en ce que** 0,98 à 1,1 équivalent de toluène est utilisé par rapport à un équivalent d'acide nitrique.

11. Procédé selon les revendications 1 à 10, **caractérisé en ce que** 1,01 à 1,05 équivalent de toluène est utilisé par rapport à un équivalent d'acide nitrique.

12. Procédé selon les revendications 1 à 11, **caractérisé en ce que** la composition des déchets acides à la sortie du réacteur s'élève à 54 à 67% d'acide sulfurique, 7 à 22% d'acide phosphorique ainsi que 18 à 27% d'eau.

13. Procédé selon les revendications 1 à 12, **caractérisé en ce que** la concentration est effectuée dans un vaporisateur à une pression de 30 à 300 mbars et une température de 100 à 200°C.

14. Procédé selon les revendications 1 à 13, **caractérisé en ce que** la concentration est effectuée dans un vaporisateur en cascade.

15. Procédé selon les revendications 1 à 14, **caractérisé en ce que** le vaporisateur fonctionne avec une partie d'épuisement.

16. Procédé selon les revendications 1 à 15, **caractérisé en ce que** la température de réaction est de l'ordre de 20 à 80°C.

17. Procédé selon les revendications 1 à 16, **caractérisé en ce qu'**une addition de 2000 ppm maximum de verre soluble peut être effectuée en vue d'empêcher l'attaque des matériaux mis en oeuvre par le composant acide mixte.
